# FASCICULE DE BREVET EUROPEEN

(11) **EP 1 334 185 B1**
(45) Date de publication et mention de la délivrance du brevet: **31.08.2011**
(21) Numéro de dépôt: 01982525.6
(22) Date de dépôt: 19.10.2001
(51) Int. Cl.: C12N 13/00, C12N 1/06, C12P 21/00

(54) **PROCEDE DE PRODUCTION DE PROTEINES PAR ELECTROPULSATION**
VERFAHREN ZUR HERSTELLUNG VON PROTEINEN DURCH ELEKTROPULSIERUNG
METHOD FOR PRODUCING PROTEINS BY ELECTROPORATION

(30) Priorité: 19.10.2000 FR 0013415
(43) Date de publication de la demande: 13.08.2003
(73) Titulaire: CENTRE NATIONAL DE LA RECHERCHE SCIENTIFIQUE (CNRS), 75794 Paris Cedex 16 (FR)
(72) Inventeur: TEISSIE, Justin, F-31520 Ramonville (FR); GANEVA, Valentina, 1421 Sophia (BG); GALUTZOV, Bojidar, 1421 Sophia (BG)
(74) Mandataire: Vaillant, Jeanne
(86) Numéro de dépôt international: PCT/FR2001/003261
(87) Numéro de publication internationale: WO 2002/033065

(56) Documents cités:
- EP-A- 0 218 352
- WO-A-00/63355
- WO-A-98/54306
- GANEVA VALENTINA ET AL: "Electropulsation as an alternative method for protein extraction from yeast." FEMS MICROBIOLOGY LETTERS, vol. 174, no. 2, 15 mai 1999 (1999-05-15), pages 279-284, XP001010438 ISSN: 0378-1097 cité dans la demande
- ROLS M-P ET AL: "HIGHLY EFFICIENT TRANSFECTION OF MAMMALIAN CELLS BY ELECTRIC FIELD PULSES APPLICATION TO LARGE VOLUMES OF CELL CULTURE BY USING A FLOW SYSTEM" EUROPEAN JOURNAL OF BIOCHEMISTRY, vol. 206, no. 1, 1992, pages 115-121, XP008012369 ISSN: 0014-2956
- QIN B -L ET AL: "INACTIVATING MICROORGANISMS USING A PULSED ELECTRIC FIELD CONTINUOUS TREATMENT SYSTEM" IEEE TRANSACTIONS ON INDUSTRY APPLICATIONS, vol. 34, no. 1, 1998, pages 43-49, XP000766888 ISSN: 0093-9994
- TEISSIE J ET AL: "ELECTROFUSION OF LARGE VOLUMES OF CELLS IN CULTURE PART I. ANCHORAGE-DEPENDENT STRAINS" BIOELECTROCHEMISTRY AND BIOENERGETICS, vol. 19, no. 1, 1988, pages 49-58, XP008012317 ISSN: 0302-4598
- BONNAFOUS PIERRE ET AL: "The generation of reactive-oxygen species associated with long-lasting pulse-induced electropermeabilisation of mammalian cells is based on a non-destructive alteration of the plasma membrane." BIOCHIMICA ET BIOPHYSICA ACTA, vol. 1461, no. 1, 9 novembre 1999 (1999-11-09), pages 123-134, XP002226825 ISSN: 0006-3002

## Description

La présente invention concerne un procédé de production de protéines d'intérêt.

Les microorganismes que sont les bactéries, champignons, cellules végétales, cellules d'insectes et cellules animales, et notamment les levures, peuvent produire des protéines, il est toutefois difficile de récupérer certains des produits de ces microorganismes, et en particulier de récupérer les macromolécules telles que les protéines.

Pour extraire des protéines d'intérêt de levure, qui sont des protéines naturellement produites, il est connu d'utiliser, d'une part, des méthodes mécaniques, d'autre part, des méthodes chimiques.

Lorsque l'on utilise les méthodes mécaniques usuelles que constituent le broyage et la lyse sous pression, il est impossible de ne pas détruire les vacuoles des levures, et la libération des protéases contenues dans les vacuoles pose des problèmes de pureté et donc de purification du produit fini.

Par ailleurs, les méthodes chimiques mènent également à la destruction des vacuoles et utilisent des détergents ou des agents de protéolyse qui s'ajoutent aux produits chimiques utilisés, comme contaminants du milieu. Ceci mène donc aussi à des problèmes de purification ultérieurs.

De plus, certains des agents chimiques inactivent les enzymes.

La présente invention concerne une approche nouvelle où un traitement d'électropulsation est appliqué sur une suspension de cellules en écoulement, suivi d'une incubation dans un milieu adapté. Les cellules peuvent être des levures, mais aussi des bactéries auxquelles peut être appliquée l'électropulsation d'une suspension dans de l'eau pure, par exemple, suivie d'une incubation relativement longue en milieu salin, et ce sans qu'aucun pré-traitement de la culture bactérienne visant à la modifier mécaniquement, chimiquement ou biologiquement n'ait eu lieu. Le traitement peut également être adapté aux cellules de mammifères, ce qui est décrit à titre d'information dans la presente demande et ne fait pas partie de la présente invention

La présente invention concerne un procédé de production de protéines dans lequel un flux de milieu liquide comportant au moins une levure ou une bactérie, en suspension à une concentration comprise entre 10⁶ et 5x10⁹ cellules/ml dans le milieu liquide, est soumis à électropulsation, l'électropulsation étant réalisée avec des intensités du champ d'au moins 1kV/cm, la durée des impulsions est supérieure à environ 0,01ms et la durée du traitement d'électropulsation étant de 0,01 à 100 s, l'étape d'électropulsation est suivie d'une étape d'incubation, et les protéines libérées sont récupérées.

Comme cela apparaîtra par la suite, on entend par électropulsation un procédé où les cellules sont soumises à un train d'impulsions électriques.

Ainsi la présente invention résout les problèmes évoqués ci-dessus. En effet, selon l'invention, le traitement électrique ne provoque pas la désintégration des cellules et on n'obtient donc pas de fragments cellulaires dans le milieu, après traitement. Tout le contenu de la cellule n'est pas libéré : les organites ainsi que la plupart des grosses protéines cytoplasmiques sont retenus par la paroi cellulaire. Les vacuoles ne sont pas affectées et les protéases ne sont pas libérées. La purification des protéines d'intérêt en est simplifiée et le procédé de l'invention permet une plus grande sélectivité des produits libérés.

Des travaux portant sur l'électropulsation en lit fixe ont été effectués, mais les conditions décrites V. Ganeva, B. Galutzov FEMS Microbiology Letters 174 (1999) 279-284 ne permettent pas la réalisation du présent procédé avec les résultats escomptés.

En effet, le procédé en lit fixe ne permet pas la perméabilisation irréversible de toutes les cellules traitées. Ainsi, la demanderesse a constaté que certaines conditions de mise en oeuvre, dans le procédé en lit fixe, pouvaient présenter certains inconvénients en flux.

Il s'agit notamment des conditions relatives à la conductivité du milieu d'électropulsation. Enfin, les traitements d'électropulsation appliqués provoquent des effets différents sur les levures, selon qu'ils ont lieu en lit fixe d'une part, et en flux d'autre part, et notamment leurs effets sur le niveau de perméabilisation des cellules.

Par ailleurs, l'inactivation des enzymes peut être évitée dans le procédé en flux, qui permet d'éviter l'échauffement notable de la suspension cellulaire. Ceci présente un grand intérêt notamment du point de vue du rendement en évitant une étape ultérieure de refroidissement et l'utilisation des dispositifs nécessaires à cette étape.

Ainsi, selon l'invention, le traitement d'une suspension des cellules à haute concentration permet d'obtenir une libération optimale sans provoquer d'échauffement du milieu.

Enfin, il est possible d'appliquer le processus selon l'invention sur des volumes importants, et d'obtenir de grandes quantités de protéines d'intérêt à un degré de pureté impossible à obtenir jusqu'ici par d'autres méthodes, et en particulier sans l'ajout d'inhibiteurs de protéase, en utilisant des milieux de composition simple.

Dans le procédé de l'invention, l'application d'un champ électrique externe de haute densité sur les cellules, et notamment les cellules en suspension, provoque l'apparition d'un potentiel qui s'ajoute au potentiel transmembranaire. Lorsqu'un certain seuil critique est dépassé, la membrane cellulaire devient perméable. Quand les cellules sont en mouvement (en flux), leur position par rapport aux électrodes change. Les impulsions auront comme cible différentes régions de la surface. Il en résulte une plus large surface perméabilisée et un niveau local du changement structurel différent que celui des cellules en statique (en batch). A des paramètres définis du champ électrique appliqué, comme l'intensité, la durée ou le nombre des impulsions, les changements de la membrane provoqués par les pulsations peuvent devenir irréversibles et il s'ensuit une libération du contenu intracellulaire.

Pour les levures qui ont une paroi, cet efflux est contrôlé par la porosité de la paroi, la perméabilité pour les différentes molécules dépendant de leur dimension.

Les inventeurs ont observé que les membranes des bactéries se perméabilisent pour des valeurs du champ électrique similaires aux valeurs employées pour les levures.

La perméabilisation est un processus très rapide qui se développe pendant les quelques millisecondes de l'application des impulsions électriques, et demeure pendant un laps de temps allant de quelques minutes à des heures après le traitement électrique.

On a pu constater que le traitement imposé aux *levures* peut provoquer la libération de l'enzyme invertase, située dans l'espace périplasmique. Il apparaît que la paroi cellulaire voit sa porosité augmenter, en plus de l'influence du champ sur la membrane plasmique.

Le procédé selon l'invention permet l'efflux et ainsi la libération, et la récupération ultérieure de protéines secrétées par la cellule et qui s'accumulent dans l'espace périplasmique.

Selon d'autres aspects, l'invention concerne également les protéines obtenues par le procédé décrit ici et les compositions en comportant.

Du point de vue des cellules que l'on peut traiter par le procédé d'invention, toutes les espèces susceptibles de produire des protéines hétérologues ou homologues peuvent être traitées, les exemples ci-après n'étant pas limitatifs.

Pour les levures, on peut citer notamment Saccharomyces, Kluyveromices, Pichia, Candida et Hansenula.

Du point de vue des bactéries, on peut citer entre autres des bactéries Gram-négatives, et notamment E.coli.

Du point de vue des cellules de mammifères, on peut citer entre autres des fibroblastes, et notamment des cellules d'hamsters chinois.

Selon l'invention, les cellules sont mises en culture, elles sont alors lavées pour éliminer les ions du milieu, puis elles subissent le traitement électrique et sont mises à incuber dans un milieu d'incubation. Ces cellules sont alors séparées du surnageant. La purification des protéines est réalisée selon les moyens usuels.

Selon la présente invention, l'étape d'électropulsation est précédée d'une étape de culture. La croissance peut y être maintenue jusqu'à la phase exponentielle ou jusqu'à la phase stationnaire. De préférence, la phase de culture est exponentielle mais des résultats positifs ont été obtenus aussi en phase stationnaire. Le milieu de culture peut donc être choisi pour permettre une expression maximum de la protéine recherchée qu'elle soit homologue ou hétérologue. La Demanderesse a mis en évidence que l'efficacité de l'électroextraction n'est pas influencée par la composition du milieu de culture.

Le transfert des cellules du milieu de culture au milieu de pulsation s'effectue après une centrifugation des suspensions.

De préférence, les cellules sont en suspension dans le milieu liquide soumis à électropulsation. La concentration de levures ou de bactéries en suspension dans le milieu liquide d'électropulsation est comprise entre environ 10⁶ et 5x10⁹ cellules/ml, de préférence entre environ 5x10⁸ et environ 2x10⁹ cellules/ml. La concentration en cellules de mammifères est de préférence environ 50 fois plus faible.

Du point de vue des conditions du traitement d'électropulsation, celui-ci peut être réalisé avec des intensités du champ d'au moins 1 kV/cm, notamment 1 et 10 kVkm, de préférence comprise entre 1 et 5 kV/cm, et notamment 2 et 4 kV/cm.

La durée des impulsions est choisie pour être supérieure à 0,01 ms, notamment supérieure à 1 ms. Elle peut atteindre jusqu'à 100 ms. Elle est de préférence choisie entre environ 0,5 et 15 ms, notamment 1 et 15 ms.

Lorsqu'on applique des champs électriques de haute densité, on applique en général une seule impulsion. On peut aussi appliquer une série d'impulsions consécutives, ladite série ayant une durée allant de quelques micros à quelques millisecondes. Ainsi, le traitement appliqué à chaque cellule dure environ de 0,01 à 100 s, de préférence de 0,15 à 15 s.

Le nombre d'impulsions reçues par chaque cellule de levure, de bactérie ou de mammifère est de l'ordre de 1 à 100, de préférence entre 5 et 20, de façon préférée entre 12 et 20, par exemple de l'ordre de 10.

Par exemple, dans le cas de 2 Hz, la durée de l'impulsion serait de 3 ms et l'intensité du champ de 3 kV/cm, dans le cas de 6 Hz, la durée est de 2 ms et l'intensité de 3,2 kV/cm, ou de 1 ms pour 4,3 kV/cm.

Comme cela apparaîtra dans les exemples, on peut ainsi appliquer les impulsions en série, et par exemple une série d'une quinzaine d'impulsions, une impulsion pouvant durer de 0,1 à 4 ms, notamment de 2 à 4 ms.

En ce qui concerne, la fréquence d'impulsions, elle est de préférence selon l'invention supérieure à 0,1 Hz, de préférence 1 Hz. Elle peut atteindre 100 Hz, voire 500 Hz. De façon plus particulièrement préférée, elle est comprise entre 1 et 100 Hz, notamment 1 et 10 Hz. Par ailleurs, le produit de la durée de chaque impulsion par la fréquence doit rester inférieur à 1.

En ce qui concerne les cellules de mammifères, l'intensité du champ électrique appliquée doit être suffisante pour permettre la sortie des protéines cytoplasmiques. Il faut, dans le cas des paramètres électriques illustrés dans l'exemple, utiliser des intensités de champ électriques supérieures à 1kV/cm pour qu'il ait libération des protéines cytoplasmiques dans le milieu extérieur. Il a été remarqué que la viabilité des cellules est de 50% dans les conditions de meilleur relargage (1,25 kV/cm).

Pour ce qui concerne le profil des impulsions de champ électrique, on peut envisager des vagues carrées, trapézoïdales, triangulaires, sinusoïdales, sinusoïdales redressées mono et bialternance, ou à déclin exponentiel, unipolaires, bipolaires, symétriques, asymétriques ; on utilise de préférence des vagues carrées.

Selon l'invention, le milieu d'électropulsation a une conductivité basse, de préférence inférieure à 2 mS/cm. Il est ainsi choisi pour limiter l'amplitude de l'effet Joule associé aux impulsions électriques. Il peut donc être un milieu salin simple. Le milieu d'électropulsation peut aussi être de l'eau déionisée, pour les levures et bactéries.

Après le traitement au champ électrique, le milieu liquide comportant les cellules traitées est mis à incuber pour favoriser la fuite des protéines d'intérêt.

Le milieu d'incubation peut être le même que le milieu de pulsation mais il peut être également modifié. La transition du milieu de pulsation à celui d'incubation peut être réalisée par transfert des cellules pulsées dans un tampon contenant un stabilisateur osmotique.

Comme milieu d'incubation, on peut utiliser selon l'invention, un milieu salin simple, comportant au moins un sel, par exemple un phosphate de potassium. Ainsi on peut utiliser un milieu d'incubation comportant au moins un tampon comme le phosphate de potassium à raison de 10 mM à 0,2 M, de préférence 50-150 mM, un stabilisateur osmotique comme le glycérol à raison de 0,05 à 1 M, de préférence 0,2-0,5 M, il peut aussi comporter un réducteur comme du dithiothreitol à raison de 0,01 à 0,2 mM -à titre d'agent conservant la stabilité des protéines extraites- on peut utiliser aussi d'autres produits contenant des groupes thiol (sulfhydrique) comme du mercaptoéthanol ou de la mercaptoéthylamine.

Le glycérol est souvent utilisé comme protecteur osmotique mais dans des concentrations de 10 à 20%. Dans le présent procédé, sa présence vise à la préservation des vacuoles intactes, sa concentration est beaucoup plus basse (par exemple 2,8%), ce qui facilite la purification ultérieure des protéines libérées.

A la fin de l'incubation, on peut purifier les protéines par tout moyen connu de séparation des protéines d'un milieu liquide, et par exemple par centrifugation de volume fixe, ou en flux continu, filtration, décantation ou précipitation, de préférence centrifugation ou filtration.

Pour ce qui est du choix des milieux de culture, d'incubation et d'extraction, notamment le spécialiste du traitement des levures, des bactéries et des cellules mammifères pourra adopter la composition de ces milieux au cas d'espèce.

Pour les levures et bactéries, le milieu soumis à l'électropulsation peut être de l'eau pure et le milieu de culture peut être salin. Pour les cellules de mammifères, le milieu de culture doit être équilibré du point de vue osmotique, de préférence, et le milieu de culture peut être similaire au milieu d'électropulsation. Ainsi on peut utiliser un milieu à faible contenu ionique, où du saccharose par exemple assure la protection contre le choc osmotique.

Le procédé de l'invention peut être appliqué avec les levures à l'extraction d'enzymes cytoplasmiques telles que l'ADH (alcool déshydrogénase), PGK (phosphoglycérate kinase), HK (hexokinase), GAPDH (glycéraldéhyde phosphate déshydrogénase), GLR (glutathion réductase), SOD (superoxyde dismutase) ainsi que la béta-galactosidase, dans le cas de Kluiveromyces lactis.

Selon l'invention, avec les levures les différentes étapes de culture, incubation et extraction peuvent notamment avoir des durées de l'ordre de :
- culture jusqu'à la phase exponentielle : environ 15h ;
- culture jusqu'à la phase stationnaire : 24 à 48h ;
- séparation des cellules du milieu de culture, lavage et dilution : 1 h ;
- traitement électrique de chaque volume traversant la chambre de pulsation : quelques secondes ;
- incubation visant la libération de protéines après électropulsation pour obtenir un maximum : entre 3 et 8h ;
- séparation des protéines : 10 mn de centrifugation à 2000xg.

Selon l'invention, avec les bactéries les différentes étapes de culture, incubation et extraction peuvent notamment avoir des durées de l'ordre de :
- préculture pendant la nuit ;
- culture jusqu'à la phase exponentielle de 4 à 10 heures ;
- séparation des cellules du milieu de culture, lavage et dilution : 1h ;
- traitement électrique de chaque volume traversant la chambre de pulsation : quelques secondes ;
- incubation visant la libération de protéines après électropulsation pour obtenir un maximum : entre 2 et 8h ;
- séparation des protéines : 10 mn de centrifugation à 2000xg.

Avec les cellules de mammifères, les différentes étapes de culture, incubation et extraction peuvent notamment avoir des durées de l'ordre de :
- culture jusqu'à la phase exponentielle de plusieurs jours pour avoir une biomasse suffisante ;
- séparation des cellules du milieu de culture, lavage et dilution : 1h;
- traitement électrique de chaque volume traversant la chambre de pulsation : quelques secondes ;
- incubation visant la libération de protéines après électropulsation pour obtenir un maximum : environ 30 min;
- séparation des protéines : 10 mn de centrifugation à 100xg.

Du point de vue du matériel mis en oeuvre pour la réalisation du procédé, une chambre d'électropulsation relie un réservoir où les cellules après leur cultivation, lavage et dilution sont aspirées par la chambre, l'écoulement les faisant passer entre les électrodes connectées à un électropulsateur. On peut adapter la chambre et matériel d'électropulsation : les impulsions électriques peuvent être appliquées à une fréquence qui est fonction de la vitesse d'écoulement, si bien que les cellules reçoivent un nombre prédéfini d'impulsions adaptées en intensité et en durée, par exemple 2 à 5 kV/cm et d'une durée de 0,1 à 15 ms, par exemple de 1 à 15 ms.

Pour des durées similaires, des intensités plus faibles de 0,5 à 5 kV/cm, de préférence 1 à 4 kV/cm, peuvent être utilisées pour les cellules de mammifères.

A la sortie de la chambre d'écoulement, le flux des cellules est dilué dans le milieu d'incubation et la fuite transmembranaire des protéines peut avoir lieu. Les protéines sont alors séparées des levures, bactéries ou cellules de mammifères par centrifugation ou filtration. Elles sont alors purifiées.

La détection de la sortie des protéines du microorganisme peut être obtenue par le test au bleu de coomassie, qui permet une évaluation globale des protéines présentes dans l'extrait, méthode de référence pour faire un bilan des protéines solubles dans un extrait soluble. Dans les exemples réalisés, les protéines membranaires ont sédimenté lors de la centrifugation. Elles ne sont plus présentes dans l'extrait.

Les enzymes peuvent être concentrées et purifiées ultérieurement par des méthodes connues. La séparation des enzymes libérées des cellules du surnageant est une étape usuelle avant de procéder à une purification ultérieure. Dans le cas de l'électropulsation, comme il n'y a pas de fragmentation des cellules, une centrifugation à 1500-2000 xg est suffisante pour les levures et les bactéries, et à 100 x g pour les cellules de mammifères. A un échelon industriel, cette séparation peut être réalisée par centrifugation en continu ou par filtration.

La mise en oeuvre du procédé d'électropulsation a été réalisée, sur des flux continus de culture cellulaire dans les conditions suivantes.

### EXEMPLES DE PRODUCTION DE PROTÉINES PAR ÉLECTROPULSATION DE LEVURES :

Exemple a) *Saccharomyces cerevisiae* (souche FY-86) a été cultivé en milieu YPD (10 g/l d'extrait de levure, 20 g/l de peptone, 20 g/l de glucose) à 30°C sous 240 rpm. La croissance y a été maintenue jusqu'à la phase exponentielle et jusqu'à la phase stationnaire.

Après la culture des levures, les cellules ont été séparées du milieu de culture par centrifugation à 2000 g pendant 5 mn, lavées deux fois avec de l'eau déionisée et diluées en eau déionisée jusqu'à une concentration de 10⁹ cellules/ml.

Les paramètres de pulsation ont été choisis de la façon suivante : une série de quinze impulsions, chacune d'une durée de 3 ms, à une fréquence de 4 Hz, le débit étant de 3 ml/mn.

Juste après le traitement, la suspension a été diluée de 5 fois dans le milieu d'extraction, qui est également dans le cas présent le milieu d'incubation. Il comportait une concentration finale de tampon de phosphate de potassium (0,1 M), du glycérol (0,3 M) et 1 mM DTT.

Le séjour dans le milieu d'extraction a été de l'ordre de 6 heures. Le processus d'électropulsation et l'incubation suivante visant l'extraction se sont déroulés à température ambiante.

L'activité des protéines récupérées a alors été évaluée par un test spécifique. La totalité des protéines libérées a été évaluée par le test Biorad.

Pour *Saccharomyces cerevisiae,* on a constaté que la libération de 80% des protéines cytoplasmiques, telles que de la glycéraldéhyde phosphate déshydrogénase, de l'hexokinase, de la phosphoglycérate kinase, dans un milieu comprenant du phosphate de potassium (0,084 M), du glycérol (0,24 M) et 0,8 mM DTT, a duré 4 heures.

Exemple b) On a réalisé un essai similaire, dans les mêmes conditions que ci-dessus, la croissance étant maintenue en phase stationnaire. On a obtenu des résultats similaires à ceux de a).

Exemple c) *Kluyveromyces lactis* (souche strain 2209 a été cultivée en milieu YPL (10 g/l d'extrait de levure, 20 g/l de peptone, 20 g/l de lactose) et dans les mêmes conditions.

Un protocole très similaire à celui de a) a été suivi.

Après la culture des levures, les cellules ont été séparées par centrifugation à 2000 g pendant 5 mn, lavées deux fois avec de l'eau déionisée et diluées en eau déionisée, jusqu'à une concentration de 10⁹ cellules/ml.

Les paramètres de pulsation ont été choisis de la façon suivante : une série de quinze impulsions, chacune d'une durée de 3 ms, à une fréquence de 4 Hz, donc à un débit pouvant atteindre 3 mi/mn. Une augmentation du débit peut être obtenue par une simple modification des caractéristiques géométriques de la chambre de pulsation et des performances de l'électropulsateur.

Juste après leur traitement, la suspension est diluée de 5 fois dans le milieu d'extraction, qui est également dans le cas présent le milieu d'incubation, et comporte une concentration initiale de tampon de phosphate de potassium (0,1 M), du glycérol (0,3 M) et 1 mM DTT. Le séjour dans le milieu d'extraction est de l'ordre de 8 heures. Le processus d'électropulsation et l'incubation suivante visant l'extraction se sont déroulés à température ambiante.

Pour *Kluyveromyces lactis,* la libération de la beta-galactosidase cytoplasmique dans un milieu (Phosphate 0,084 M), du glycérol (0,24 M) et 1,6 mM DTT, a duré 7 à 8 heures.

Après l'incubation, les cellules sont séparées du liquide contenant les protéines libérées par centrifugation.

Exemple d) : dans les mêmes conditions, on a reproduit l'exemple a) avec des paramètres de pulsation suivants : une série de 15 impulsions, chacune d'une durée de 3 ms, à une fréquence de 50 Hz, le débit étant de 60 ml/mn.

Exemple e) : on a reproduit l'exemple c), à ceci près que les paramètres de pulsations ont été choisis de la façon suivante : une série de 15 impulsions, chacune d'une durée de 3 ms, à une fréquence de 6 Hz, donc un débit pouvant atteindre 7,2 ml/mn.

Ces données sont comparées avec les résultats d'une lyse classique des levures : on a constaté pour a), comme pour b), c), d) et e) l'absence de dégradation protéolytique, alors qu'elle a lieu avec les autres procédés. Moins de 5 à 10 % des enzymes ont été désactivées. Ce résultat montre la supériorité de la méthode électrique par rapport à ce qui est actuellement utilisé à l'échelle du laboratoire ou en processus industriel.

Les profils après électrophorèse sur gel de polyacrylamine (PAGE) où des bandes nettes sans traînée sont présentes, ont confirmé l'absence de dégradation protéolytique au cours du processus d'extraction.

Les résultats quantitatifs suivants ont été obtenus pour a), comme pour b), c), d) et e).
- le bilan des protéines récupérées représente de 45 à 50% de toutes les protéines cellulaires (qui peuvent être obtenues par lyse cellulaire enzymatique ou désintégration mécanique) ;
- le bilan des enzymes libérées et analysées est : phosphoglycérate kinase, glycéraldéhyde phosphate déshydrogénase et hexokinase de l'ordre de 80 à 90% de leur contenu dans les cellules.

L'activité spécifique des enzymes dans le surnageant des cellules pulsées est de 1,7 à 2 fois supérieure que celle obtenue par broyage mécanique ou lyse enzymatique des cellules. On constate donc au stade de l'incubation, qu'une purification des enzymes fonctionnelles récupérées a lieu.

### EXEMPLES DE PRODUCTION DE PROTÉINES PAR ÉLECTROPULSATION DE BACTÉRIES

### - Culture

50 ml d'une culture de nuit d'E coli (BL 21) ont été resuspendus dans 450 ml de milieu LB. La culture a été incubée pendant 2 h à 37°C sous agitation.

La culture présentait une densité optique de E₆₆₀ = 0.7. Elle a été centrifugée à 4 000 rpm pendant 10 min. Le culot a été resuspendu en 500 ml d'eau milliQ (Millipore). Une incubation de 30 min à la température ambiante (25°C) a suivi.

On a appliqué une centrifugation à 4 000 rpm pendant 10 min et le culot a été resuspendu dans 45 ml d'eau milliQ. Les deux lavages successifs ont permis d'éliminer une grande partie des ions du milieu où se trouvent les bactéries.

### - Conditions de traitement:

La chambre de pulsation présente un volume de 0.3 ml et une distance de 3mm entre les électrodes (planes parallèles, en acier inoxydable).

Le débit a été fixé à 2,4 ml/min par une pompe péristaltique.

Des trains d' impulsions de durée individuelle de 2 ms à la fréquence de 3 Hz ont été appliqués en continu. Cela correspond à l'application de 22 impulsions à chaque bactérie.

La tension du générateur a été fixée à 1500 V, soit une intensité de champ de 5 kV/cm.

Après le traitement électrique, 100 microlitres de la suspension bactérienne électrotraitée ont été resuspendus dans 400 microlitres de tampon PBS 0.105 M, pH=7. L'échantillon a alors été soumis à une incubation à 37°C.

Les cellules contrôles ont été traitées de la même façon mais la tension du générateur est fixée à 0 V.

Après une incubation de 5 h, les cellules ont été centrifugées dans une centrifugeuse Eppendorf pour 3 min (13000 x g). On a déterminé le contenu de protéines dans le surnageant.

### - Détermination des protéines

Un bilan global des protéines présentes dans le surnageant a alors été évalué par un test colorimétrique.

L'échantillon a été évalué en mesurant la densité optique à 595 nm du mélange de 100 microlitres de surnageant, 700 microlitres d'eau milliQ et 200 microlitres de réactif Bradford (Biorad, USA).

La référence du spectrophotomètre est le mélange de 800 microlitres d'eau milliQ et de 200 microlitres de réactif de Bradford.

Une gamme de référence de protéines a été réalisée avec de l'albumine bovine (Sigma, USA)

En conclusion, on a déterminé que le surnageant de l'échantillon essai possédait une densité optique de 0,570 soit 11,4 µg de protéine, alors que celui du contrôle était de 0,103 soit 2,4 µg de protéine. Dans ces conditions de mesure, 9 µg de protéines ont été extraites de l'échantillon de bactéries évalué par le traitement électrique suivi d'une incubation de 5h.

La quantité de protéines (9 µg) correspondait au volume de l'échantillon soit 0,1 ml de surnageant, soit 25 µl de la suspension bactérienne électrotraitée.

Aucun prétraitement de la culture bactérienne visant à modifier mécaniquement, chimiquement ou biologiquement n'a eu lieu.

### EXEMPLE DE PRODUCTION DE PROTÉINES PAR ÉLECTROPULSATION DE CELLULES DE MAMMIFÈRES

### -Culture:

Les cellules de hamsters chinois (CHO, clone WTT) sont des cellules partiellement transformées. Elles ont été cultivées à 37°C en suspension dans des flacons en verre du type spinner maintenus sous agitation douce (100 rpm) pour éviter l'adhésion des cellules au support. Le milieu de culture utilisé était le milieu minimum essentiel de Eagle: MEM 0111 (Eurobio) complémenté avec, 8% de sérum foetal de veau (Seromed), des antibiotiques (pénicilline à 100 unités par ml, streptomycine à 100 µg par ml), de la L-glutamine (0,58 mg par ml).

### - Electropulsation

Lors de l'électropulsation des cellules, le milieu de culture a été remplacé par du milieu de pulsation. Pour les cellules CHO, il est constitué de tampon phosphate (10mM, pH 7,2) de saccharose (250mM) et de MgCl₂ (1 mM) ((Conductance 1400µS/cm±200µS/cm; osmolarité ∼ 0,317osmol/kg)). Ce milieu iso-osmotique et de faible force ionique permet de limiter, grâce à sa faible conductance l'effet Joule associé aux impulsions électriques.

Les cellules ont été centrifugées à 100 xg pendant 5 minutes. Le surnageant a été éliminé puis le culot a été repris par du milieu de pulsation.

Les cellules ont été électropulsées à champs variables par 10 impulsions d'1ms, délivrées à la fréquence d' 1 Hz. Le débit de l'écoulement est 1,2ml/min. Dans la chambre, les électrodes étaient constituées par 2 lames parallèles séparées par une distance interélectrode de 0,4cm. Le volume de la chambre de pulsation était de 0,2ml, et la direction du champ perpendiculaire à celle de l'écoulement moyen.

Après l'électropulsation, les cellules ont été mises à température ambiante pour permettre aux membranes des cellules de retrouver leur état natif. 1,5 ml de suspension cellulaire (1.10⁶cellules/ml) ont été récupérés pour chaque essai. Les cellules ont été incubées 10 minutes à température ambiante puis placées à 4°C. Les cellules ont ensuite été centrifugées pendant 5 minutes à 100xg (800 rpm, centrifugeuse C500, Jouan). Le surnageant (∼1ml) a été prélevé et conservé à 4°C.

### - Dosage

Le dosage des protéines a été réalisé dans du tampon de pulsation avec le kit Biorad. Une gamme étalon est réalisée en parallèle en utilisant de la Sérum Albumine Bovine (BSA, 1mg/ml). Une solution diluée de BSA (0,1 mg/ml) a servi à préparer les différentes dilutions de la gamme étalon. 800µl des solutions de la gamme étalon et des différents essais ont été additionnés à 200µl de réactif de Biorad. La lecture de la densité optique (DO) a été réalisée à l'aide d'un spectrophotomètre à 595nm. On obtient un bilan global des protéines électroextraites.

### - Bilan

Les résultats sur la libération de protéines intracellulaires en fonction de l'intensité du champ électrique E (kV/cm) ont montré que pour une intensité de champ de 1,25 kV/cm, la concentration de protéines libérées est de 25 µg/ml et 50% des cellules restent viables. La viabilité est évaluée 24h après le traitement électrique par la technique de coloration au crystal violet. L'ensemencement des boites de culture pour quantifier la viabilité *a été* réalisé avec le même nombre de cellules (∼0,75.10⁶ cellules/ml). Le 100% de viabilité est exprimé par rapport à la DO obtenue à 595nm pour des cellules n'ayant subi aucun traitement électrique mais ayant été traitées en flux continu.

## Revendications

1. Procédé de production de protéines dans lequel un flux de milieu liquide comportant au moins une levure ou une bactérie, en suspension à une concentration comprise entre 10⁶ et 5x10⁹ cellules/ml dans le milieu liquide, est soumis à électropulsation, l'électropulsation étant réalisée avec des intensités du champ d'au moins 1 kV/cm, la durée des impulsions est supérieure à environ 0,01 ms et la durée du traitement d'électropulsation étant de 0,01 à 100 s, l'étape d'électropulsation est suivie d'une étape d'incubation, et les protéines libérées sont récupérées.

2. Procédé selon la revendication 1, **caractérisé en ce que** la concentration en levure(s) ou en bactérie(s) en suspension dans le milieu liquide est comprise entre 5x10⁸ et 5x10⁹ cellules/ml.

3. Procédé selon la revendication 1 ou 2, **caractérisé en ce que** la levure est choisie parmi les espèces Saccharomyces, Kluyveromyces, Pichia, Candida et Hansenula.

4. Procédé selon l'une des revendications 1 à 3, **caractérisé en ce que** les protéines libérées sont séparées des levures ou bactéries par filtration ou centrifugation.

5. Procédé selon l'une des revendications 1 à 4, **caractérisé en ce que** l'intensité du champ est comprise entre 1 et 10 kV/cm.

6. Procédé selon l'une des revendications 1 à 5, **caractérisé en ce que** l'intensité du champ est comprise entre 1 et 5kV/cm.

7. Procédé selon l'une des revendications 1 à 6, **caractérisé en ce que** la durée des impulsions est inférieure à 100 ms.

8. Procédé selon l'une des revendications 1 à 7, **caractérisé en ce que** la durée des impulsions est comprise entre 0,5 et 15 ms.

9. Procédé selon l'une des revendications 1 à 8, **caractérisé en ce que** la durée du traitement d'électropulsation est de 0,15 à 15 s.

10. Procédé selon l'une des revendications 1 à 9, **caractérisé en ce que** la fréquence des impulsions est supérieure à 0,1 Hz.

11. Procédé selon l'une des revendications 1 à 10, **caractérisé en ce que** la fréquence des impulsions est supérieure à 1 Hz.

12. Procédé selon l'une des revendications 1 à 10, **caractérisé en ce que** la fréquence des impulsions est comprise entre 1 et 500 Hz.

13. Procédé selon l'une des revendications 1 à 12, **caractérisé en ce que** le nombre d'impulsions reçues par chaque cellule de levure ou de bactérie est compris entre 1 et 100.

14. Procédé selon l'une des revendications 1 à 13, **caractérisé en ce que** le nombre d'impulsions reçues par chaque cellule est compris entre 5 et 20.

15. Procédé selon l'une des revendications 1 à 14, **caractérisé en ce que** le nombre d'impulsions reçues par chaque cellule de levure ou de bactérie est de l'ordre de 10.

16. Procédé selon l'une des revendications 1 à 15, **caractérisé en ce que** le milieu d'incubation comporte au moins un sel.

17. Procédé selon l'une des revendications 1 à 16, **caractérisé en ce que** le milieu d'incubation comporte du phosphate de potassium.

18. Procédé selon l'une des revendications 1 à 17, **caractérisé en ce que** le milieu d'extraction comporte au moins un agent préservant la stabilité des protéines extraites.

19. Procédé selon l'une des revendications 1 à 18, **caractérisé en ce que** le milieu d'extraction comporte au moins un agent réducteur.

20. Procédé selon l'une des revendications 1 à 19, **caractérisé en ce que** le milieu d'extraction comporte du dithiothréitol.

21. Procédé selon l'une des revendications 1 à 20, **caractérisé en ce que** le milieu d'électropulsation est de l'eau déionisée.

22. Procédé selon l'une des revendications 1 à 20, **caractérisé en ce que** le profil des impulsions de champ électrique est en vagues carrées, trapézoïdales, triangulaires, sinusoïdales, sinusoïdales redressées mono et bialternance, ou à déclin exponentiel, unipolaires, bipolaires, symétriques ou asymétriques.

23. Procédé selon l'une des revendications 1 à 21, **caractérisé en ce que** le profil des impulsions est en vagues carrées.

## Claims

1. A process for producing proteins in which a stream of liquid medium comprising at least one yeast or a bacterium, in suspension in the liquid medium at a concentration in the range 10⁶ to 5 x 10⁹ cells/ml, undergoes electropulsing, the electropulsing being carried out with field intensities of at least 1kV/cm, the pulse duration is higher than about 0,01ms and the electropulsing treatment duration is in the range 0,01 to 100s, the electropulsing step is followed by an incubation step, and the proteins that are liberated are recovered.

2. A process according to claim 1, **characterized in that** the concentration of yeast/s or bacterium/a in suspension in the liquid medium is in the range 5 x 10⁸ to 5 x 10⁹ cells/ml.

3. A process according to claim 1 or 2, **characterized in that** the yeast is selected from species Saccharomyces, Kluyveromyces, Pichia, Candida and Hansenula.

4. A process according to one of claims 1 to 3, **characterized in that** the liberated proteins are separated from the yeasts or bacteria by filtering or centrifuging.

5. A process according to one of claims 1 to 4, **characterized in that** the field intensity is in the range 1 to 10 kV/cm.

6. A process according to one of claims 1 to 5, **characterized in that** the field intensity is in the range 1 to 5 kV/cm.

7. A process according to one of claims 1 to 6, **characterized in that** the pulse duration is less than 100 ms.

8. A process according to one of claims 1 to 7, **characterized in that** the pulse duration is in the range 0.5 to 15 ms.

9. A process according to one of claims 1 to 8, **characterized in that** the electropulsing treatment duration is in the range 0.15 to 15 s.

10. A process according to one of claims 1 to 9, **characterized in that** the pulse frequency is more than 0.1 Hz.

11. A process according to one of claims 1 to 10, **characterized in that** the pulse frequency is more than 1 Hz.

12. A process according to one of claims 1 to 10, **characterized in that** the pulse frequency is in the range 1 to 500 Hz.

13. A process according to one of claims 1 to 12, **characterized in that** the number of pulses received by each yeast or bacterium cell is in the range 1 to 100.

14. A process according to one of claims 1 to 13, **characterized in that** the number of pulses received by each yeast or bacterium cell is in the range 5 to 20.

15. A process according to one of claims 1 to 14, **characterized in that** the number of pulses received by each yeast or bacterium cell is of the order of 10.

16. A process according to one of claims 1 to 15, **characterized in that** the incubation medium comprises at least one salt.

17. A process according to one of claims 1 to 16, **characterized in that** the incubation medium comprises potassium phosphate.

18. A process according to one of claims 1 to 17, **characterized in that** the extraction medium comprises at least one agent that maintains the stability of the extracted proteins.

19. A process according to one of claims 1 to 18, **characterized in that** the extraction medium comprises at least one reducing agent.

20. A process according to one of claims 1 to 19, **characterized in that** the extraction medium comprises dithiothreitol.

21. A process according to one of claims 1 to 20, **characterized in that** the electropulsing medium is deionized water.

22. A process according to one of claims 1 to 20, **characterized in that** the profile of the electric field pulses is a square, trapezoidal, triangular, sinusoidal, single or half-wave rectified sinusoidal wave, or a wave with an exponential decay, unipolar, bipolar, symmetrical or asymmetrical.

23. A process according to one of claims 1 to 21, **characterized in that** the pulse profile is a square wave.

## Patentansprüche

1. Verfahren zur Herstellung von Proteinen, wobei ein Strom eines wässrigen Milieus, der wenigstens eine Hefe oder ein Bakterium in Suspension in einer Konzentration umfasst, die zwischen 10⁶ und 5x10⁹ Zellen/ml in dem wässrigen Milieu enthalten ist, einer Elektropulsierung unterworfen wird, wobei die Elektropulsierung mit Feldstärken von wenigstens 1kV/cm durchgeführt wird, die Impulsdauer über etwa 0,01 ms liegt und die Dauer der Elektropulsierungsbehandlung 0,01 bis 100 s beträgt, die Elektropulsierungsstufe von einer Inkubationsstufe gefolgt wird und die freigesetzten Proteine gewonnen werden.

2. Verfahren gemäß Anspruch 1, **dadurch gekennzeichnet, dass** die Konzentration an Hefe(n) oder an Bakterium/Bakterien in Suspension in dem wässrigen Milieu zwischen 5x10⁸ und 5x10⁹ Zellen/ml enthalten ist.

3. Verfahren gemäß Anspruch 1 oder 2, **dadurch gekennzeichnet, dass** die Hefe unter den Spezien Saccharomyces, Klyeveromyces, Pichia, Candida und Hensenula ausgewählt ist.

4. Verfahren gemäß einem der Ansprüche 1 bis 3, **dadurch gekennzeichnet, dass** die freigesetzten Proteine von den Hefen oder Bakterien durch Filtration oder Zentrifugation abgetrennt werden.

5. Verfahren nach einem der Ansprüche 1 bis 4, **dadurch gekennzeichnet, dass** die Feldstärke zwischen 1 und 10 kV/cm enthalten ist.

6. Verfahren nach einem der Ansprüche 1 bis 5, **dadurch gekennzeichnet, dass** die Feldstärke zwischen 1 und 5 kV/cm enthalten ist.

7. Verfahren nach einem der Ansprüche 1 bis 6, **dadurch gekennzeichnet, dass** die Impulsdauer unter 100 ms liegt

8. Verfahren nach einem der Ansprüche 1 bis 7, **dadurch gekennzeichnet, dass** die Impulsdauer zwischen 0,5 und 15 ms enthalten ist.

9. Verfahren nach einem der Ansprüche 1 bis 8, **dadurch gekennzeichnet, dass** die Dauer der Elektropulsierungsbehandlung 0,15 bis 15 s beträgt.

10. Verfahren nach einem der Ansprüche 1 bis 9, **dadurch gekennzeichnet, dass** die Impulsfrequenz über 0,1 Hz liegt.

11. Verfahren nach einem der Ansprüche 1 bis 10, **dadurch gekennzeichnet, dass** die Impulsfrequenz über 1 Hz liegt.

12. Verfahren nach einem der Ansprüche 1 bis 10, **dadurch gekennzeichnet, dass** die Impulsfrequenz zwischen 1 und 500 Hz liegt.

13. Verfahren nach einem der Ansprüche 1 bis 12, **dadurch gekennzeichnet, dass** die von jeder Hefe- oder Bakterienzelle empfangene Impulszahl zwischen 1 und 100 enthalten ist.

14. Verfahren nach einem der Ansprüche 1 bis 13, **dadurch gekennzeichnet, dass** die von jeder Hefe- oder Bakterienzelle empfangene Impulszahl zwischen 5 und 20 enthalten ist.

15. Verfahren nach einem der Ansprüche 1 bis 14, **dadurch gekennzeichnet, dass** die von jeder Hefe- oder Bakterienzelle empfangene Impulszahl die Größenordnung 10 hat.

16. Verfahren nach einem der Ansprüche 1 bis 15, **dadurch gekennzeichnet, dass** das Inkubationsmilieu wenigstens ein Salz umfasst.

17. Verfahren nach einem der Ansprüche 1 bis 16, **dadurch gekennzeichnet, dass** das Inkubationsmilieu Kaliumphosphat umfasst.

18. Verfahren nach einem der Ansprüche 1 bis 17, **dadurch gekennzeichnet, dass** das Extraktionsmilieu wenigstens ein Mittel umfasst, das die Stabilität des extrahierten Proteins konserviert.

19. Verfahren nach einem der Ansprüche 1 bis 18, **dadurch gekennzeichnet, dass** das Extraktionsmilieu wenigstens ein Reduktionsmittel umfasst.

20. Verfahren nach einem der Ansprüche 1 bis 19, **dadurch gekennzeichnet, dass** das Extraktionsmilieu Dithiothreitol umfasst.

21. Verfahren nach einem der Ansprüche 1 bis 20, **dadurch gekennzeichnet, dass** das Elektropulsierungsmilieu entionisiertes Wasser ist.

22. Verfahren nach einem der Ansprüche 1 bis 20, **dadurch gekennzeichnet, dass** das Impulsprofil des elektrischen Feldes die Form von viereckigen, trapazförmigen, dreieckigen, sinusförmigen, mono- oder bialternierend verstärkten sinusförmigen oder exponentiell, unipolar, bipolar, symmetrisch oder asymmetrisch geneigten Wellen aufweist.

23. Verfahren nach einem der Ansprüche 1 bis 21, **dadurch gekennzeichnet, dass** das Impulsprofil eine viereckige Welle ist.
